# EUROPÄISCHE PATENTSCHRIFT

(11) **EP 1 440 049 B1**
(45) Veröffentlichungstag und Bekanntmachung des Hinweises auf die Patenterteilung: **06.09.2006**
(21) Anmeldenummer: 02801897.6
(22) Anmeldetag: 18.10.2002
(51) Int. Cl.: C07C 29/88, C07C 31/22

(54) **VERFAHREN ZUR ZERSETZUNG VON AMMONIUMFORMIATEN IN POLYOLHALTIGEN REAKTIONSGEMISCHEN**
METHOD FOR THE DECOMPOSITION OF AMMONIUM FORMATES IN REACTION MIXTURES CONTAINING POLYOL
PROCEDE DE DECOMPOSITION DE FORMIATES D'AMMONIUM DANS DES MELANGES DE REACTION CONTENANT DES POLYOLS

(30) Priorität: 24.10.2001 DE 10152525
(43) Veröffentlichungstag der Anmeldung: 28.07.2004
(73) Patentinhaber: BASF Aktiengesellschaft, 67056 Ludwigshafen (DE)
(72) Erfinder: KOCH, Michael, 68163 Mannheim (DE); WARTINI, Alexander, 69120 Heidelberg (DE); SIRCH, Tilman, 67105 Schifferstadt (DE); DERNBACH, Matthias, 69221 Dossenheim (DE)
(86) Internationale Anmeldenummer: PCT/EP2002/011671
(87) Internationale Veröffentlichungsnummer: WO 2003/035594

(56) Entgegenhaltungen:
- EP-A- 0 289 921
- DE-A- 3 340 791
- DE-A- 19 848 569

## Beschreibung

Die Erfindung bezieht sich auf das Gebiet der industriellen organischen Chemie. Genauer gesagt betrifft die vorliegende Erfindung ein Verfahren zur effektiven Zersetzung von in Methylolalkanen enthaltenem Trialkylammoniumformiat, das aus dem bei der Methylolalkanal-Herstellung als Katalysator benutzten Trialkylamin und der als Nebenprodukt gebildeten Ameisensäure entsteht.

Die Kondensation von Formaldehyd mit CH-aciden höheren Alkanalen zu Methylolalkanalen, im allgemeinen Dimethylol- und Trimethylolalkanalen und Überführung der erhaltenen Verbindungen in Polyole ist ein weit verbreitetes Verfahren in der Chemie. Beispiele für derart erhaltene, wichtige Triole sind Trimethylolpropan, Trimethylolethan und Trimethylolbutan, die zu Herstellung von Lakken, Urethanen und Polyestern ein breites Verwendungsgebiet gefunden haben. Weitere wichtige Verbindungen sind Pentaerythrit, erhältlich durch Kondensation von Formaldehyd und Acetaldehyd, sowie Neopentylglykol aus iso-Butyraldehyd und Formaldehyd. Der vierwertige Alkohol Pentaerythrit wird ebenfalls häufig in der Lackindustrie eingesetzt, hat aber auch eine große Wichtigkeit bei der Herstellung von Sprengstoffen erlangt.

Die erwähnten Polyole können nach verschiedenen Verfahren hergestellt werden. Eine Methode ist das sogenannte Cannizzaro-Verfahren, das noch weiterhin unterteilt wird in das anorganische und das organische Cannizzaro-Verfahren. Bei der anorganischen Variante setzt man einen Überschuß Formaldehyd mit dem entsprechenden Alkanal in Gegenwart von stöchiometrischen Mengen einer anorganischen Base wie NaOH oder Ca(OH)₂ um. Das in der ersten Stufe gebildete Methylolalkanal reagiert in der zweiten Stufe mit dem überschüssigen Formaldehyd in einer Disproportionierungsreaktion zu dem entsprechenden Polyol und dem Formiat der entsprechenden Base, also etwa Natrium- oder Calciumformiat.

Beim organischen Cannizzaro-Verfahren wird anstelle der anorganischen Base ein teritäres Amin, generell ein Trialkylamin, eingesetzt. Die Reaktion verläuft wie oben dargelegt, wobei ein Äquivalent Ammoniumformiat des entsprechenden Amins gebildet wird. Dies kann durch entsprechende Maßnahmen weiter aufgearbeitet werden, wodurch zumindest das Amin wiedergewonnen und in die Reaktion zurückgeführt werden kann. Das erhaltene Roh-Polyol kann auf verschiedene Weise zu reinem Polyol aufgearbeitet werden.

Eine Weiterentwicklung ist das Hydrierverfahren, in dem ein entsprechendes Alkanal und Formaldehyd nicht in Gegenwart von mindestens stöchiometrischen Mengen, sondern von katalytischen Mengen eines teritären Amins, im allgemeinen ca. 5 bis 10 Mol-%, miteinander zur Reaktion gebracht werden. Dabei bleibt die Reaktion auf der Stufe von 2,2-Dimethylolalkanal stehen, das anschließend durch Hydrierung in Trimethylolalkan überführt wird. Die Beschreibung des effektiven Verfahrens findet sich in der WO 98/28253 der Anmelderin.

Verschiedene Varianten dieses Hydrierverfahrens sind unter anderem beschrieben in den Anmeldungen DE-A-25 07 461, DE-A-27 02 582, DE-A-28 13 201 und DE-A-33 40 791.

Beim Hydrierverfahren fallen zwar vorteilhafterweise keine stöchiometrischen Mengen des Formiates an wie beim organischen Cannizzaro-Verfahren, dennoch wird Trialkylammoniumformiat als Produkt einer in sehr geringem Umfang als Nebenreaktion ablaufenden gekreuzten Cannizzaro-Reaktion gebildet.

Trialkylammoniumformiate reagieren unter bestimmten Bedingungen, beispielsweise den Entwässern oder Erhitzen von enthaltenden Trimethylolalkan-Lösungen, zu Trialkylamin und Trimethylolpropanformiaten. Diese schmälern die Ausbeute an Trimethylolalkan und können nur schwer ohne unerwünschte Abbaureaktion gespalten werden. Es bestand daher Interesse an der Abtrennung der Trialkylammoniumformiate.

DE 198 48 569 offenbart ein Verfahren zur Zersetzung von Formiaten tertiärer Amine, die als Nebenprodukte in durch das organische Cannizzaro-Verfahren hergestellten Trimethylolalkan-Lösungen enthalten sind. Diese Formiate werden, vorzugsweise in Gegenwart von modifizierten Edelmetallkatalysatoren und erhöhtem Druck, durch Erhitzen in Wasserstoff und Kohlendioxid und/oder Wasser und Kohlenmonoxid und das tertiäre Amin zersetzt. Die Formiatumsätze bei diesem Verfahren sind unbefriedigend, zudem wird die Bildung weiterer Nebenprodukte beobachtet.

Das vorstehend genannte Verfahren eignet sich zudem nur bedingt zur effektiven Aufarbeitung eines nach dem sogenannten Hydrierverfahren enthaltenen Trimethylolalkan-Gemisches, in dem lediglich katalytische Mengen an Trialkylamin verwendet werden und das somit auch nur geringe Mengen an Trialkylammoniumformiat enthält. Es ist daher die Aufgabe der vorliegenden Erfindung, ein Verfahren bereitzustellen, das sowohl zur Aufarbeitung von nach dem Hydrierverfahren als auch nach dem organischen Cannizzaro-Verfahren erhaltener Gemische geeignet ist. Dieses Verfahren sollte es weiterhin ermöglichen, Trimethylolalkane mit hoher Reinheit und niedriger Farbzahl, bevorzugt kleiner 10 Apha herzustellen.

Diese Aufgabe wird gelöst durch ein Verfahren zum Entfernen von Trialkylammoniumformiat aus Methylolalkanen, die durch Kondensation von Formaldehyd mit einem höheren Aldehyd erhalten wurden, bei 100 bis 250°C und einem Druck oberhalb von 10⁶ Pa, wobei dieses Verfahren dadurch gekennzeichnet ist, dass Trialkylammoniumformiat bei erhöhter Temperatur an Katalysatoren, die mindestens ein Metall der Gruppen 8 bis 12 des Periodensystems enthalten unter Zufuhr von Wasserstoff enthaltendem Gas zersetzt wird.

Für die Aufreinigung durch das erfindungsgemäße Verfahren geeignete Methylolalkane sind beispielsweise Neopentylglykol, Pentaerythrit, Trimethylolpropan, Trimethylolbutan, Trimethylolethan, 2-Ethyl-1,3-propandiol, 2-Methyl-1,3-propandiol, Glycerin, Dimethylolpropan, Dipentaerythrit und 1,1-, 1,2-, 1,3- und 1,4-Cyclohexandimethanol.

Im Rahmen des erfindungsgemäßen Verfahren werden bevorzugt Trimethylolalkane, die nach dem organischen Cannizzarro oder dem Hydrierverfahren hergestellt werden, von Trialkylammoniumformiaten gereinigt. Bevorzugt werden nach dem Hydrierverfahren hergestellte Trimethylolalkane, besonders bevorzugt Trimethylolpropan, nachfolgend TMP abgekürzt, gereinigt.

Die Herstellung von Trialkylammoniumformiat-enthaltendem Roh-TMP nach dem Cannizzaro-Verfahren ist beispielsweise in DE 198 48 569 offenbart.

Das TMP wird nach dem Hydrierverfahren erhalten durch Kondensation von n-Butyraldehyd mit Formaldehyd in Gegenwart von katalytischen Mengen eines tertiären Amins und anschließender katalytischer Hydrierung des entstandenen Dimethylolbutanal-Gemisches. Dieses Roh-TMP enthält keine Alkali- oder Erdalkaliformiate oder sonstige Verunreinigungen, die bei dem anorganischen Cannizzaro-Verfahren entstehen. Ebenfalls enthält das Roh-TMP nur geringe Mengen, ca. 5 bis 10 Mol-% an Trialkylammoniumformiaten bzw. freiem Trialkylamin, anders als beim organischen Cannizzaro-Verfahren.

Das der Hydrierung entstammende und dem erfindungsgemäßen Reinigungsverfahren zu unterwerfende Roh-TMP enthält neben Trimethylolpropan und Wasser noch Methanol, Trialkylamin, Trialkylammoniumformiat, längerkettige lineare und verzweigte Alkohole und Diole, beispielsweise Methylbutanol oder Ethylpropandiol, Additionsprodukte von Formaldehyd und Methanol an Trimethylolpropan, Acetale wie Dimethylolbutyraldehyd-TMP-Acetal sowie das sogenannte Di-TMP.

Gute Ergebnisse wurden erzielt mit Hydrierausträgen, die 10 bis 40 Gew.-% Trimethylolpropan, 0 bis 10 Gew.-% 2,2-Dimethylolbutanal, 0,5 bis 5 Gew.-% Methanol, 0 bis 6 Gew.-% Methylbutanol, 1 bis 10 Gew.-% Trialkylammoniumformiat, 0 bis 5 Gew.-% 2-Ethylpropandiol; 0,1 bis 10 Gew.-% Hochsieder wie Di-TMP oder auch andere Additionsprodukte und 5 bis 80 Gew.-% Wasser aufweisen. Hydrierausträge einer derartigen Zusammensetzung können beispielsweise erhalten werden nach dem in der WO 98/28253 beschriebenen Verfahren. Es ist möglich den so erhaltenen Hydrieraustrag entsprechend den Beispielen 2 und 3 der DE-A-199 63 435 vor der erfindungsgemäßen Aufreinigung zur Zersetzung des Trialkylammoniumformiates zunächst durch kontinuierliche Destillation aufzuarbeiten. Bevorzugt ist jedoch die erfindungsgemäße Aufreinigung der Hydrierausträge ohne vorherige destillative Behandlung.

Als Katalysatoren finden in dem erfindungsgemäßen Verfahren heterogene Katalysatoren, die mindestens ein Metall der 8. bis 12. Gruppe des Periodensystems, beispielsweise Ruthenium, Osmium, Iridium, Platin, Palladium, Rhodium, Eisen, Kupfer, Kobalt, Nickel und Zink sowie Kombinationen dieser Metalle, enthalten. Diese Metalle können sowohl in Form der reinen Metalle als auch von deren Verbindungen, beispielsweise Oxyden oder Sulfiden, eingesetzt werden. Vorzugsweise werden Kupfer-, Nickel-, Kobalt-, Ruthenium- oder Palladiumkatalysatoren verwendet. Diese Katalysatoren können auf den üblichen Trägern, beispielsweise TiO₂, Al₂O₃, ZrO₂, SiO₂, Kohle oder deren Gemischen, aufgebracht sein. Die so erhaltenen, geträgerten Katalysatoren können in allen bekannten Konfektionierungsformen vorliegen. Beispiele sind Stränge oder Tabletten.

Die Verwendung geträgerter Kupfer-, Nickel- und/oder Cobalt- enthaltender Katalysatoren ist bevorzugt.

Für die Verwendung im Verfahren gemäß der vorliegenden Erfindung sind Raney-Kupfer, Raney-Nickel und Raney-Cobalt-Katalysatoren geeignet. Diese Raney-Katalysator können in allen bekannten Konfektionierungsformen, beispielsweise als Tabletten, Stränge oder Granulat vorliegen. Geeignete Raney-Kupfer-Katalysatoren sind beispielsweise die Raney-Kupfer-Katalysatoren in Form von Nuggets, die aus der WO 99/03801 bekannt sind, auf die hier ausdrücklich Bezug genommen wird. Diese Katalysatoren weisen eine Korngröße der Nuggets von 2 bis 7 mm, ein Kupfergehalt von 40 bis 90 Gew.-%, eine Oberfläche nach Langmuir von 5 bis 50 m²/g, eine Kupferoberfläche von 0,5 bis 7m²/g, ein Hg-Porenvolumen von 0,01 bis 0,12 ml/g und einen mittleren Porendurchmesser von 50 bis 300 nm auf.

Aus EP-A-672 452, auf die hier ausdrücklich Bezug genommen wird, sind geeignete nickelhaltige Katalysatoren bekannt, die 65 bis 80 % Nickel, berechnet als Nickeloxid, 10 bis 25 % Silicium, berechnet als Siliciumdioxid, 2 bis 10 % Zirkonium, berechnet als Zirkoniumoxid und 0 bis 10 % Aluminium, berechnet als Aluminiumoxid enthalten mit der Maßgabe, daß die Summe aus dem Gehalt an Siliciumdioxid und Aluminiumoxid mindestens 15 % beträgt (Prozentangaben in Gew.-%, bezogen auf die Gesamtmasse des Katalysators), die durch Zugabe einer sauren wäßrigen Lösung von Nickel-, Zirkonium- und gewünschtenfalls Aluminiumsalzen zu einer basischen wäßrigen Lösung von Silicium- und gewünschtenfalls Aluminiumverbindungen, wobei der pH-Wert auf mindestens 6,5 abgesenkt wird und anschließend durch Zugabe weiterer basischer Lösung auf 7 bis 8 eingestellt wird, Isolieren des so ausgefällten Feststoffs, Trocknen, Formen und Calcinieren erhältlich sind.

Weiterhin können die aus EP-A-044 444, auf die hier ausdrücklich Bezug genommen wird, bekannten Hydrierkatalysatoren mit einer spezifischen Oberfläche von 50 bis 150 m²/g, die ganz oder teilweise Spinellstruktur aufweisen, bei denen Kupfer in Form von Kupferoxid enthalten ist und bei deren Herstellung Kupfer und Aluminium in einem Verhältnis von 0,25 bis 3 Atomen Kupfer zu einem Atom Aluminium aus ihren Verbindungen in Gegenwart von Carbonaten bei einem pH von 4,5 bis 9 gefällt werden und der so erhaltene Niederschlag bis 300 bis 800°C calciniert wird.

Geeignet für die Verwendung in dem erfindungsgemäßen Verfahren ist auch der aus DE-A 198 26 396 bekannte Zirkonium, Kupfer, Cobalt und Nickel enthaltende Katalysator, der frei von sauerstoffhaltigen Verbindungen des Molydäns ist.

Gemäß einer besonders bevorzugten Ausführungsform wird die erfindungsgemäße Aufreinigung in Gegenwart des aus der DE-A 198 09 418, auf die hier ausdrücklich Bezug genommen wird, bekannten Katalysators, der einen anorganischen Träger, der TiO₂ enthält, und als Aktivkomponente Kupfer oder ein Gemisch aus Kupfer mit mindestens einem der Metalle, ausgewählt aus der Gruppe Zink, Aluminium, Cer, einem Edelmetall und einem Metall der VIII. Nebengruppe, umfasst und dessen spezifische Kupferoberfläche maximal 10 m²/g beträgt, durchgeführt. Diese Katalysatoren weisen bevorzugt als Träger TiO₂ oder eine Mischung aus TiO₂ und Al₂O₃ oder eine Mischung aus TiO₂ und ZrO₂ oder eine Mischung aus TiO₂, Al₂O₃ und ZrO₂ auf, besonders bevorzugt wird TiO₂ verwendet.

Bei der Herstellung dieses Katalysators gemäß DE-A 19809418 kann metallisches Cu-Pulver als weiteres Additiv während der Tablettierung zugesetzt werden, dass die Kupferoberfläche maximal 10 m²/g beträgt.

In einer besonderen Ausführungsform der Erfindung wird die Zersetzung des im Roh-TMP enthaltenen Trialkylammoniumformiates an einem zur Hydrierung der Vorstufe des TMP's (2,2-Dimethylolbutanal) geeigneten Katalysator durchgeführt, beispielsweise an dem aus DE-A-198 09 418 bekannten Kupferkatalysator.

Diese Ausführungsform ist besonders wirtschaftlich, da die Zersetzung des Trialkylammoniumformiate im Hydrierreaktor des Hydrierverfahrens gemäß WO 98/28253 erfolgen kann und nur ein Katalysator benötigt wird. Die Zersetzungsprodukte des Trialkylammoniumformiates, CO und Wasser und/oder CO₂ und Wasserstoff können über die Abgasleitung aus dem Reaktor entfernt werden. Die Zersetzung der Trialkylammoniumformiate nach dem erfindungsgemäßen Verfahren kann jedoch ebenso in einen gesonderten Reaktor durchgeführt werden.

Im Rahmen des erfindungsgemäßen Verfahrens wird die Zersetzung der Trialkylammoniumformiate im allgemeinen bei einer Temperatur von 100 bis 250°C, vorzugsweise 140 bis 220°C durchgeführt. Die dabei verwendeten Drücke liegen in der Regel bei oberhalb von 10⁶ Pa, bevorzugt 2·10⁶ bis 15·10⁶ Pa.

Das erfindungsgemäße Verfahren kann entweder kontinuierlich oder diskontinuierlich durchgeführt werden, wobei die kontinuierliche Verfahrensdurchführung bevorzugt ist.

Bei der kontinuierlichen Verfahrensführung beträgt die Menge des Roh-Trimethylolalkans aus dem Hydrierverfahren bzw. dem organischen Cannizzaro-Verfahren vorzugsweise ungefähr 0,05 bis ungefähr 3 kg pro Liter Katalysator pro Stunde, weiter bevorzugt ungefähr 0,1 bis ungefähr 1 kg pro Liter Katalysator pro Stunde.

Als Hydriergase können beliebige Gase verwendet werden, die freien Wasserstoff enthalten und keine schädlichen Mengen an Katalysatorgiften, wie beispielsweise CO, aufweisen. Beispielsweise können Reformerabgase verwendet werden. Vorzugsweise wird reiner Wasserstoff verwendet.

Im folgenden soll das erfindungsgemäße Verfahren anhand von Ausführungsbeispielen näher erläutert werden.

### Beispiele

### Herstellung Roh-TMP

Eine Apparatur bestehend aus zwei beheizbaren, durch Überlaufrohre miteinander verbundenen Rührkesseln mit einem Fassungsvermögen von insgesamt 72 l wurde mit frischer, wässriger Formaldehydlösung (4300 g/l in Form der 40 %igen wässrigen Lösung und n-Butyraldehyd (1800 g/h) und mit frischem Trimethylamin als Katalysator (130 g/h) in Form der 45 %igen wäßrigen Lösung kontinuierlich beschickt. Die Reaktoren wurden dabei auf 40°C temperiert.

Der Austrag wurde direkt in den oberen Teil eines Fallfilmverdampfers mit aufgesetzter Kolonne (11 bar Heizdampf) geleitet und dort bei normalem Druck destillativ in ein leichtsiedendes Kopfprodukt, im wesentlichen enthaltend n-Butyraldehyd, Ethylacrolein, Formaldehyd, Wasser und Trimethylamin, und ein hochsiedendes Sumpfprodukt aufgetrennt.

Das Kopfprodukt wurde kontinuierlich kondensiert und in die oben beschriebenen Reaktoren zurückgeführt.

Das hochsiedende Sumpfprodukt aus dem Verdampfer (ca. 33,5 kg/h) wurde kontinuierlich mit frischem Trimethylamin-Katalysator (50 g/h, in Form der 45%igen wässrigen Lösung) versetzt und in einen beheizbaren, mit Füllkörpern versehenen Rohrreaktor mit einem Leervolumen von 12 1 geführt. Der Reaktor war dabei auf 40°C temperiert.

Der Austrag des Nachreaktors wurde kontinuierlich in den oberen Teil einer weiteren Destillationseinrichtung, der Formaldehydabtrennung (11 bar Heizdampf), gegeben und dort destillativ in ein leichtsiedendes Kopfprodukt, im wesentlichen enthaltend Ethylacrolein, Formaldehyd, Wasser und Trimethylamin, und ein hochsiedendes Sumpfprodukt aufgetrennt. Das leichtsiedende Kopfprodukt (27 kg/h) wurde kontinuierlich kondensiert und in den ersten Rührkessel zurückgeleitet, wohingegen das hochsiedende Sumpfprodukt gesammelt wurde.

Das so erhaltene Sumpfprodukt enthielt neben Wasser im wesentlichen Dimethylolbutyraldehyd, Formaldehyd und Spuren Monomethylolbutyraldehyd. Er wurde dann einer kontinuierlichen Hydrierung unterworfen. Dazu wurde die Reaktionslösung bei 90 bar und 115°C in einem Hauptreaktor in Kreislauf/Rieselfahrweise und einem nachgeschalteten Nachreaktor in Kreislauffahrweise hydriert. Der Katalysator wurde analog J der DE 198 09 418 hergestellt. Er enthielt 40 % CuO, 20 % Cu und 40% TiO₂. Die verwendete Apparatur bestand aus einem 10 m langen beheizten Hauptreaktor (Innendurchmesser: 27 mm) und einem 5,3 m langen beheizten Nachreaktor (Innendurchmesser: 25 mm). Der Kreislaufdurchsatz betrug 25 l/h Flüssigkeit, der Reaktorzulauf wurde auf 4 kg/h eingestellt. Dementsprechend wurden 4 kg/h Hydrieraustrag erhalten.

### Beispiele 1 bis 4

Das verwendete TMP hat die Zusammensetzung 25 Gew.-% TMP, 0,35 Gew.-%- 2,2-Dimethylolbutanal, 0,53 Gew.-% Methanol, 0,078 Gew.-% Methylbutanol, 0,23 Gew.-% Ethylpropandiol, 0,43 Gew.-% Addukten von TMP mit Formaldehyd und Methanol, 0,036% TMP-Formiat, 0,46 Gew.-% TMP-Dimethylbutanal-Acetale, 0,46 Gew.-% Hochsieder, 1,7 Gew.-% Trimethylammoniumformiat und 70 Gew.-% Wasser. Von dieser Rohlösung wurden 180 ml in Gegenwart eines bei 180°C und 25 bar vorreduzierten Katalysators gemäß Tabelle 1 mit Wasserstoff bei 180°C und 90 bar behandelt. Nach einer Stunde wurden der Dimethylolbutanal-Gehalt und der TMP-Gehalt gaschromatographisch ermittelt. Die Formiatkonzentration wurde mit Hilfe einer Titration mit Tetrabutylammoniumhydroxid ermittelt. Die erzielten Ergebnisse sind in Tabelle 1 zusammengefasst.

### Vergleichsbeispiel 5

Es wurde Verfahren wie in den Beispielen 1 bis 4 jedoch wurde die Umsetzung in Abwesenheit eines Katalysators durchgeführt. Das Ergebnis ist der Tabelle zu entnehmen.

| Nr. | Katalysator | Formkörper | Menge Katalysator [g] | DMB Fl.-%¹ | TMP Fl.-%¹ | Formiat Gew.-%² | Formiat umsatz [%] |
|---|---|---|---|---|---|---|---|
| Einsatzstoff | | | | 1,17 | 83,4 | 0,74 - | |
| 1 | Cu/TiO₂ (DE 198 09 418) | 3x3 mm Tabletten | 20 | 0 | 85,1 | 0,33 | 55 |
| 2 | Cu/Al₂O₃ (EP 0 044 444) | 5x5 mm Tabletten | 9,5 | 0 | 85,2 | 0,40 | 46 |
| 3 | Ni/SiO₂/Al₂O₃/ZrO₂ (EP 0672 452) | 1,5 mm Stränge | 12,5 | 0 | 84,5 | 0,09 | 87 |
| 4 | Co/Ni/Cu/ZrO₂ (DE 198 26 396) | 5x3 mm Tabletten | 20,5 | 0 | 85,1 | 0,31 | 58 |
| 5 | - | - | - | 0 | 83,4 | 0,71 | 4 |

| | | | | | | | |
|---|---|---|---|---|---|---|---|
| ¹GC-Analytik (Dedektion ohne Wasser) | | | | | | | |
| ²Titration mit Tetrabutylammoniumhydroxid | | | | | | | |
| ³DMB = 2,2-Dimethylbutanal | | | | | | | |

Aus der Tabelle ist ersichtlich, dass die katalytische Zersetzung von Ammoniumformiat bei 180°C an obigen Cu-, Ni- und Ce-Katalysatoren mit hohen Umsätzen möglich ist. Demgegenüber wird unter rein thermischen Bedingungen im Vergleichsbeispiel nahezu kein Formiatumsatz erzielt. Außerdem wird deutlich, dass unter den erfindungsgemäßen Bedingungen die TMP-Ausbeute durch Hydrierung von DMB erhöht wird.

## Patentansprüche

1. Verfahren zum Entfernen von Trialkylammoniumformiat aus Methylolalkanen, die durch Kondensation von Formaldehyd mit einem höheren Aldehyd erhalten wurden, bei 100 bis 250°C und einem Druck oberhalb von 10⁶ Pa, **dadurch gekennzeichnet, dass** Trialkylammoniumformiat an Katalysatoren, die mindestens ein Metall der Gruppen 8 bis 12 des Periodensystems enthalten, unter Zufuhr von wasserstoffenthaltendem Gas zersetzt wird.

2. Verfahren nach Anspruch 1, **dadurch gekennzeichnet, dass** geträgerte Kupfer-, Nickel und/oder Kobalt enthaltende Katalysatoren verwendet werden.

3. Verfahren nach Anspruch 1 oder 2, **dadurch gekennzeichnet, dass** ein zur Hydrierung von 2,2-Dimethylolbutanal geeigneter Katalysator verwendet wird

4. Verfahren nach einem der Ansprüche 1 bis 3, **dadurch gekennzeichnet, dass** nach dem Hydrierverfahren erhaltenes Trimethylolalkan eingesetzt wird.

5. Verfahren nach einem der Ansprüche 1 bis 4, **dadurch gekennzeichnet, dass** Trimethylolpropan eingesetzt wird.

6. Verfahren nach einem der Ansprüche 1 bis 5, **dadurch gekennzeichnet, dass** es bei ein Druck von 2·10⁶ bis 15·10⁶ Pa durchgeführt wird.

7. Verfahren nach einem der Ansprüche 1 bis 6, **dadurch gekennzeichnet, dass** es im Hydrierreaktor des Hydrierverfahrens durchgeführt wird.

## Claims

1. A process for removing trialkylammonium formate from methylolalkanes obtained by condensation of formaldehyde with a higher aldehyde, at from 100 to 250°C and a pressure above 10⁶Pa, which comprises decomposing trialkylammonium formate with the addition of a hydrogen-containing gas over catalysts comprising at least one metal of groups 8 to 12 of the Periodic Table.

2. The process according to claim 1, wherein supported copper-, nickel- and/or cobalt-containing catalysts are used.

3. The process according to claim 1 or 2, wherein a catalyst suitable for the hydrogenation of 2,2-dimethylolbutanal is used.

4. The process according to any of claims 1 to 3, wherein trimethylolalkane obtained by the hydrogenation process is used.

5. The process according to any of claims 1 to 4, wherein trimethylolpropane is used.

6. The process according to any of claims 1 to 5 carried out at a pressure of from 2x10⁶ to 15x10⁶ Pa.

7. The process according to any of claims 1 to 6 carried out in the hydrogenation reactor of the hydrogenation process.

## Revendications

1. Procédé d'élimination de formiate de trialkylammonium à partir de méthylolalcanes, qui ont été obtenus par condensation de formaldéhyde avec un aldéhyde supérieur, à 100 jusqu'à 250°C et à une pression supérieure à 10⁶ Pa, **caractérisé en ce qu'**on décompose du formiate de trialkyl-ammonium sur des catalyseurs, qui contiennent au moins un métal des groupes 8 à 12 du système périodique, avec apport de gaz contenant de l'hydrogène.

2. Procédé suivant la revendication 1, **caractérisé en ce qu'**on utilise des catalyseurs contenant du cuivre, du nickel et/ou du cobalt sur support.

3. Procédé suivant la revendication 1 ou 2, **caractérisé en ce qu'**on utilise un catalyseur approprié pour l'hydrogénation de 2,2-diméthylol-butanal.

4. Procédé suivant l'une des revendications 1 à 3, **caractérisé en ce qu'**on met en oeuvre du triméthylolalcane obtenu après le procédé d'hydrogénation.

5. Procédé suivant l'une des revendications 1 à 4, **caractérisé en ce qu'**on met en oeuvre du triméthylolpropane.

6. Procédé suivant l'une des revendications 1 à 5, **caractérisé en ce qu'**il est effectué à une pression de 2·10⁶ à 15·10⁶ Pa.

7. Procédé suivant l'une des revendications 1 à 6, **caractérisé en ce qu'**il est effectué dans le réacteur d'hydrogénation du procédé d'hydrogénation.
